# FASCICULE DE BREVET EUROPEEN

(11) **EP 1 907 527 B1**
(45) Date de publication et mention de la délivrance du brevet: **03.02.2016**
(21) Numéro de dépôt: 06778860.4
(22) Date de dépôt: 11.07.2006
(51) Int. Cl.: C12M 1/34, C12M 1/12

(54) **DISPOSITIF DE PREPARATION D'UN ECHANTILLON DE FLUIDE BIOLOGIQUE EN VUE D'UNE ANALYSE BACTERIOLOGIQUE**
VORRICHTUNG ZUR PRÄPARATION EINER KÖRPERFLÜSSIGKEIT FÜR EINE BAKTERIOLOGISCHE ANALYSE
DEVICE FOR PREPARING A BODY FLUID FOR A BACTERIOLOGICAL ANALYSIS

(30) Priorité: 12.07.2005 FR 0552151
(43) Date de publication de la demande: 09.04.2008
(73) Titulaire: Hemosystem, 13006 Marseille (FR)
(72) Inventeur: BESSON-FAURE, Isabelle, F-13400 Aubagne (FR); HERMET, Jean-Pierre, F-92100 Boulogne (FR); RIBAULT, Sébastien, 67380 Lingolsheim (FR)
(74) Mandataire: von Kreisler Selting Werner - Partnerschaft von Patentanwälten und Rechtsanwälten mbB
(86) Numéro de dépôt international: PCT/FR2006/001690
(87) Numéro de publication internationale: WO 2007/006972

(56) Documents cités:
- EP-A- 0 122 581
- WO-A-01/32828
- WO-A-95/02050
- WO-A-95/04098
- FR-A- 2 853 326
- US-A- 5 858 253
- US-B2- 6 869 769

## Description

La présente invention se rapporte au domaine de la préparation d'échantillons de fluide biologique, plus particulièrement du sang, en vue de leur analyse biologique.

La présente Invention se rapporte plus particulièrement au domaine de la microbiologie et propose un procédé et un dispositif pour échantillonner et traiter un volume de sang total en vue d'une analyse bactériologique.

Le procédé et le dispositif décrits dans la présente invention sont destinés à prélever un échantillon de sang total, à réduire/éliminer les cellules eucaryotes/humaines présentes dans cet échantillon et à isoler/concentrer les cellules procaryotes/microorganismes éventuellement présents dans cet échantillon en vue de leur détection et/ou identification par des méthodes spécifiques. Parmi ces méthodes, on peut citer ① les méthodes de biologie cellulaire comme la culture sur boite de pétri, les techniques de détection directe en microscopie et ② les méthodes de biologie moléculaire d'analyse de l'ADN et de l'ARN avec, par exemple, la technique dite RFLP (restriction fragment length polymorphisms) et la technique d'amplification d'ADN ou PCR (polymerase chain reaction) avec en particulier, la méthode de PCR en temps réel.

Le sang est normalement stérile. Il est essentiel de pouvoir mettre en évidence une éventuelle bactériémie, le retard de prise en charge du patient et donc le retard de diagnostic jouant sur la fréquence des décès. La méthode permettant la détection et l'identification des bactéries présentes dans le sang est donc essentielle. A l'heure actuelle, on utilise des techniques de microbiologie classique comprenant successivement une hémoculture, une première identification visuelle après coloration de Gram puis une subculture en milieu solide pour une identification secondaire et un antibiogramme. Les résultats sont généralement obtenus sous plusieurs jours. Les autres méthodes employées de type biologie moléculaire ne permettent que des identifications précises liées à des indications particulières, par exemple la détection du streptocoque de groupe B pendant la grossesse

Les résultats des tests biologiques réalisés sur des échantillons sanguins sont très dépendants des conditions pré-analytiques. En effet, le type de prélèvement réalisé (veineux ou artériel), l'anticoagulant utilisé, le matériel à disposition influent de façon importante sur la qualité des résultats. De plus, pour une analyse bactériologique, les conditions de test doivent faire appel à des procédures strictes afin d'éviter toute contamination en cours de manipulation, amenant des résultats appelés « faux-positifs », par des microorganismes présents dans l'environnement. Ces conditions doivent être encore plus drastiques pour des analyses de biologie moléculaire qui font appel à des réactifs hautement sensibles à des interférences naturellement présentes dans l'environnement. La variabilité des conditions pré-analytiques alliée à la multiplicité des méthodes de détection/identification font du test sanguin bactériologique en biologie moléculaire un paramètre difficilement standardisable. En effet, la préparation des échantillons en vue d'une analyse bactériologique fait habituellement appel à des techniques « maison », non standardisées, à réaliser dans un environnement stérile avec du matériel spécifique à cette application. Le dispositif décrit dans la présente invention est une voie de standardisation. C'est un système clos sans risque de contamination de l'échantillon. Le prélèvement se fait directement dans le tube de prélèvement par aspiration sans ouverture de celui-ci et toutes les étapes de préparation sont réalisées au sein du dispositif sans rupture de stérilité.

Au laboratoire, la PCR permet généralement une détection par amplification de quelques copies de génome bactérien dans un échantillon. C'est une analyse rapide qui s'effectue en quelques heures et qui permet également de connaître l'espèce de la bactérie mise en cause. Cependant, l'utilisation des méthodes de biologie moléculaire est limitée en partie par la présence de nombreux facteurs dans le sang qui réduisent l'efficacité de l'amplification tels que les Immunoglobulines G dont la concentration plasmatique est de 8 à 18 g/litre, l'heme, l'hémoglobine, principal constituant des globules rouges et la lactoferrine, présente dans les leucocytes, les fortes concentrations en ADN eucaryotes venant des cellules nucléées sanguines telles que les leucocytes. De ce fait, les volumes analysés sont faibles pour limiter la quantité d'inhibiteurs : avec les méthodes actuellement disponibles sur le marché le volume d'échantillon traitable est de 100µL et il dépasse rarement les 200µL en fonction des kits. Dans le cas des bactériémies symptomatiques ou non symptomatiques la charge bactérienne est très faible et peut être Inférieure à 1 bactérie par millilitre de sang. Dans ce cas, il est statistiquement démontré que 200 µL est un volume insuffisant pour détecter ces faibles concentrations. Le dispositif décrit dans la présente invention permet de réduire les facteurs d'inhibition plasmatiques et cellulaires rendant possible une extension de volume jusqu'à 15 mL de sang total et de ce fait, une augmentation de la sensibilité de la méthode.

Afin de s'affranchir des facteurs d'inhibition cellulaire, les méthodes PCR sont classiquement effectuées sur du sérum issu de la centrifugation du sang total. En effet, au cours d'une centrifugation, les éléments figurés du sang sont concentrés dans le culot alors que les composés solubles comme ADN et ARN libres restent dans le surnageant qui est prélevé pour analyse. Ce procédé ne peut s'appliquer aux bactéries car leur densité est comparable à celles des éléments figurés du sang et elles sont également retrouvées dans le culot cellulaire.

Dans le brevet US-6 869 769, un dispositif, une méthode et un kit sont décrits pour séparer les éléments figurés du sang (leucocytes, globules rouges, plaquettes) des ARN/ADN viraux ou ADN bactériens sur support solide en vue d'une analyse PCR. Ce procédé ne peut être appliqué que sur des volumes de sang extrêmement réduits de l'ordre d'une goutte et ne peut s'appliquer à la recherche et/ou identification des microorganismes dans le cas de bactériémie où la charge en microorganismes est très faible.

Le brevet US-5 958 257 divulgue un dispositif de séparation d'un composé sanguine comprenant un récipient pourvu d'une chambre dans laquelle un piston est déplaçable entre une position d'ouverture et une position d'obturation, ladite chambre comprenant une zone de séparation et un moyen d'introduction d'un fluide dan ladite chambre, ledit moyen d'introduction étant dispose dans la partie supérieure de la chambre, et ledit piston comprenant un moyen d'obturation coopérant avec la zone de séparation de sorte à définir un volume supérieur et un volume inférieur de part et d'autre de ladite zone.

L'invention vise à pallier les inconvénients et les manques de l'état de la technique en proposant un dispositif et un procédé facilitant l'analyse rapide d'échantillon de fluide biologique dans le domaine de la microbiologie de façon efficace, standardisée, simple avec une exposition réduite de l'utilisateur vis à vis des risques biologiques. Ce procédé et ce dispositif sont particulièrement adaptés à une utilisation en routine dans le secteur hospitalier pour la préparation d'un échantillon sanguin en vue d'une analyse bactériologique en particulier en biologie moléculaire permettant au biologiste et au clinicien d'accéder rapidement à un résultat.

A cet effet et selon un premier aspect, l'invention concerne un dispositif de préparation d'un échantillon de fluide biologique en vue de son analyse bactériologique comprenant un récipient pourvu d'une chambre dans laquelle un piston est déplaçable entre une position d'ouverture et une position d'obturation, la chambre comprenant une zone de séparation et un moyen d'introduction d'un fluide dans ladite chambre, ledit moyen d'introduction étant disposé dans la partie supérieure de la chambre et le piston comprenant un moyen d'obturation coopérant avec la zone de séparation de sorte à définir un volume supérieur et un volume Inférieur de part et d'autre de ladite zone, la volume supérieur et le volume inférieur communiquant entre eux lorsque le piston est en position d'ouverture et étant isolés l'un de l'autre de façon étanche lorsque le piston est en position d'obturation, et ledit dispositif étant caractérisé en ce que la zone de séparation comprend un goulot d'étranglement, le volume inférieur s'étendant sous ledit goulot d'étranglement, le moyen d'obturation étant agencé pour boucher ledit goulot lorsque le piston est en position d'obturation de sorte à isoler le volume inférieur du volume supérieur.

Le dispositif décrit dans la présente invention est le lien entre l'échantillon de sang à tester et la plate-forme d'analyse, il fait partie d'une solution globale et standardisée de détection rapide des bactéries dans la sang. Le dispositif comprend différents volumes indépendants mais reliés pouvant contenir des solutions de réactifs prêts à l'emploi.

Durant son utilisation en hôpital, le dispositif permet de réaliser différentes étapes séquentielles et intégrées

Selon un deuxième aspect, l'invention concerne un procédé de préparation d'un échantillon de fluide biologique en vue de son analyse bactériologique utilisant un dispositif de préparation tel que décrit ci-dessus, ledit procédé comprenant les étapes consistant à :
- introduire le fluide biologique dans la chambre, le piston étant en position d'ouverture ;
- après sédimentation du fluide biologique, déplacer le piston dans sa position d'obturation de sorte à séparer le culot de sédiment du surnageant, ledit culot se trouvant dans le volume inférieur et ledit surnageant se trouvant dans le volume supérieur ;
- mélanger le surnageant avec une solution de réactifs agencée pour favoriser la croissance des microorganismes présents dans ledit surnageant ;
- prélever ledit surnageant en vue de son analyse bactériologique.

Plus particulièrement, le dispositif permet de prélever ou de recevoir, de façon stérile, un échantillon de 1 ml minimum à 15 ml maximum de sang total issu d'un tube de prélèvement sanguin standard prélevé sur anticoagulant.

Le dispositif associé au tube de prélèvement permettent le prélèvement de l'échantillon par un système d'aspiration (soufflet, poire, piston).

Le dispositif permet une incubation dans la chambre pendant un temps allant de 20 minutes à 60 heures, temps pendant lequel les globules rouges sédimentent en culot et les microorganismes se développent. Cette chambre peut être pré remplie avec une première solution de réactifs accélérant la sédimentation des globules rouges et/ou le développement des microorganismes.

Le dispositif permet de prélever ou d'isoler 6 à 20mL du surnageant résultant de l'étape précédente sans prélever le culot présent dans le volume inférieur.

Le dispositif permet le mélange de ce surnageant à une deuxième solution de réactifs dans la chambre ou après transfert dans une deuxième chambre. L'ensemble est incubé sous agitation pendant 30 minutés à 6 heures temps pendant lequel les plaquettes agrègent et les microorganismes se développent.

Le dispositif permet la filtration de la totalité de l'échantillon résultant sur un filtre seringue de porosité supérieure à 5µm à connectique « luer » (entrée femelle « luerlock », sortie mâle « luer » ou « luer-lock ») pour retenir les agrégats/amas plaquettaires et les globules blancs résiduels et, en ligne, un support de membrane à connectique d'entrée « luer-lock » femelle constitué de deux parties Imbriquées et détachables contenant une membrane filtre de 0,2µm à 1µm de porosité retenant les bactéries. Un exemple de support de membrane est décrit dans le brevet US-2004/0208796.

Les bactéries isolées sur cette dernière membrane peuvent alors subir une préparation pour une analyse en biologie moléculaire. Classiquement, un protocole de lyse physique ou chimique ou physico-chimique est appliqué pour casser les cellules et récupérer le matériel génétique à analyser. L'avantage du pré traitement de l'échantillon au moyen du procédé et dispositif décrits dans cette invention est de débarrasser les microorganismes d'intérêt de tous les facteurs d'interférence des tests, facteurs solubles plasmatiques ou facteurs cellulaires. Pour cela, les différentes chambres du dispositif peuvent contenir des solutions de réactifs prêts à l'emploi :
La première solution, dans la chambre, est composée de réactifs accélérant la sédimentation des globules rouges et la croissance des microorganismes :
   - Milieu de culture pour micro organismes ;
   - Polymères ;
   - Agglutinines ; '
   - Eau osmosée.
La deuxième solution dans une autre chambre (ou ajoutée dans la chambre du dispositif), est composée de réactifs permettant l'agrégation des plaquettes et la croissance des bactéries :
   - Milieu de culture pour micro organismes ;
   - Agent d'agrégation plaquettaire.

D'autres objets et avantages de l'invention apparaîtront au cours de la description qui suit, faite en référence aux dessins annexés.

La figure 1 est une représentation schématique de côté du dispositif selon l'invention.

La figure 2 est une représentation schématique en coupe selon l'axe A-A de la figure 1.

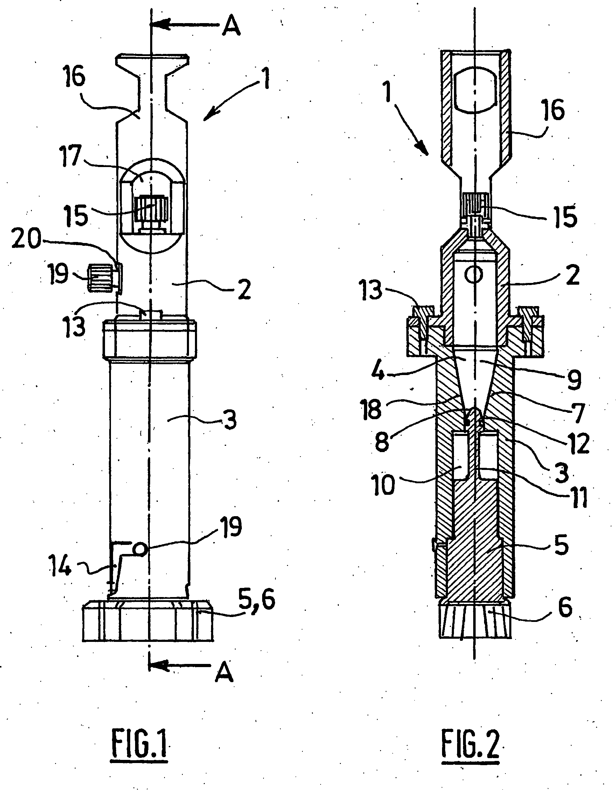

En référence aux figures, on décrit un dispositif 1 de préparation d'un échantillon de fluide biologique comportant trois pièces principales assemblées définissant un récipient. La partie supérieure 2 du dispositif est associée à la partie inférieure 3 pour former une chambre 4 fermée à sa base par un piston 5 déplaçable entre une position d'ouverture et une position d'obturation dans la partie inférieure 3 du dispositif et qui constitue également le socle 6 du dispositif. La chambre 4 comprend une zone de séparation 7. Le piston 5 comprend un moyen d'obturation 8 coopérant avec la zone de séparation 7 de sorte à définir un volume supérieur 9 et un volume inférieur 10 de part et d'autre de la zone 7. Le volume supérieur 9 et le volume inférieur 10 communique entre eux lorsque le piston 5 est en position d'ouverture et étant isolés l'un de l'autre de façon étanche lorsque le piston 5 est en position d'obturation, comme représenté sur la figure 2.

Le piston 5 comprend une tige 11 s'étendant dans le volume inférieur 10, ledit volume inférieur s'étendant de part et d'autre de la tige 11. La tige 11 comprend une protubérance 12 formant un moyen d'obturation 8 prévue à sa partie extrême libre.

Les parties supérieure 2 et inférieure 3 sont assemblées au moyen de vis 13 ou peuvent être collées. La partie inférieure 3 comprend une rainure de guidage 14 et le piston 5 comprend un ergot 19, ledit ergot étant disposé dans ladite rainure. Le piston 5 se trouve en position d'obturation lorsque l'ergot 19 est disposé à l'une des partie extrêmes de la rainure 14, comme représenté sur les figures, et en position d'ouverture lorsque l'ergot 19 est disposé à l'autre partie extrême de la rainure 14.

Avantageusement, le dispositif est en verre siliconé ou en matière plastique non adhérent pour les cellules par exemple, en polypropylène ou en polyacrylique de type polyméthacrylate de méthyle. Le matériaux du dispositif doit être compatible avec une stérilisation, de préférence par irradiation β ou y. La protubérance 12 de l'obturateur 8 est préférentiellement en élastomère de façon à assurer une obturation étanche s'adaptant au diamètre du dispositif.

Les différentes étapes du procédé en relation avec la structure du dispositif sont détaillées ci-après. Au cours de la première étape, l'échantillon est introduit par la partie supérieure 2 du dispositif par l'intermédiaire de moyens d'introduction du fluide dans la chambre 4. Les moyens d'introduction sont par exemple formé par une connexion Luer 15. Un corps de pompe équipée d'une aiguille protégée capable de percer les bouchons caoutchouc des tubes de prélèvement est vissé sur la connexion Luer 15 sur la partie supérieure du dispositif. Le corps de pompe est maintenu dans un élément de réception 16 associé à la partie supérieure du récipient. L'élément de réception est par exemple formé par une jupe de diamètre supérieur au diamètre du corps de pompe accueillant le tube. La jupe est percée de deux fenêtres 17 qui permettent la manipulation (vissage - dévissage) des objets se positionnant sur le Luer 15. Le tube de prélèvement contenant le sang est positionné bouchon vers le bas et est guidé sur l'aiguille dans le corps de pompe. En exerçant une pression verticale sur le tube, le bouchon caoutchouc est percé et l'aiguille se retrouve au contact du liquide biologique. Une aspiration du contenu du tube est créée en actionnant le piston 5 en tirant sur le socle 6. Ce piston 5 a une course définie au moyen de l'ergot qui coulisse dans la rainure 14 permettant l'aspiration du liquide. Quelques aller-retour du piston (mouvements de haut en bas) permettent le remplissage de la chambre 4 et, dans le cas de la présence d'une première solution de réactifs, le mélange du sang avec la première solution de réactifs. Le piston 5 est immobilisé en position d'ouverture au moyen de l'ergot 19 retenu dans la partie basse de la rainure 14. Le dispositif est alors posé sur son socle 6 pendant un temps allant de 20 minutes à 60 heures. Avantageusement, le dispositif est placé dans une enceinte thermorégulée entre 30°C et 37°C afin de faciliter la croissance des micro-organismes. Pendant cette période d'incubation, les globules rouges sédimentent dans le volume inférieur 10. Le culot est d'autant plus compact et irréversible que le sang a été mélangé et est incubé avec la première solution de réactifs contenant des agglutinines.

La zone de séparation 7 comprend un goulot d'étranglement 18 dont la forme facilite la sédimentation avant le passage dans le volume inférieur 10. Le volume inférieur 10 s'étend sous le goulot d'étranglement 18 et le moyen d'obturation 8 est agencé pour boucher ledit goulot 18 lorsque le piston 5 est en position d'obturation de sorte à isoler le volume inférieur 10 du volume supérieur 11. Le goulot 18 présente par exemple une forme annulaire sensiblement tronconique s'étendant dans le volume supérieur 4. Cette forme garantit une agglutination maximale avant le passage dans le volume inférieur 10.

L'utilisation d' agglutinines permet d'obtenir un culot dense avec exclusion des liquides et une perte moindre de bactéries au sein du réseau de globules rouges sédimentés. Après incubation, le piston 5 est débloqué de sa position d'ouverture par rotation du socle 6 et est délicatement poussé de façon à ce que l'ergot 19 coulisse dans la rainure 14 jusqu'à sa position d'obturation. Le piston 5 est immobilisé en position d'obturation au moyen de l'ergot 19 retenu dans la partie extrême de la rainure 14. Le piston 5 entraîne le moyen d'obturation 8 qui vient boucher de façon hermétique le goulot 18 isolant dans le volume inférieur 10 le culot de globules rouges. Le volume supérieur 9 contient la partie plasmatique, les plaquettes sanguines, les globules blancs et les bactéries. Plus le temps d'incubation est long plus la concentration en bactéries est élevée. L'étape suivante est caractérisée par l'introduction d'une deuxième solution de réactifs possédant des propriétés d'agrégation plaquettaire. Le corps de pompe positionné à la première étape sur l'élément de réception 16 est dévissé manuellement, grâce aux fenêtres 17 permettant un accès direct au corps de pompe, et éliminé. Une seringue avec embout Luer et contenant la deuxième solution de réactifs est positionnée et vissée à sa place. La solution est introduite dans le volume supérieur 9. Une agitation par retournement du dispositif est appliquée permettant à la solution d'être mélangée au surnageant issu de l'étape précédente. Le dispositif 1 est alors placé horizontalement sur un agitateur à bascule, à la perpendiculaire de l'axe d'agitation pendant 30 minutes à 6 heures. Avantageusement, l'agitateur portant le dispositif est placé dans une enceinte thermorégulée entre 30°C et 37°C afin de faciliter la croissance des micro-organismes. Pendant cette période d'incubation, les plaquettes agrègent dans le volume supérieur 9. Plus le temps d'incubation est long plus la concentration en bactéries est élevée. L'étape suivante peut être réalisée de différentes façon :

Selon une première réalisation, à la fin de l'incubation, le dispositif est retourné, piston 5 vers le haut et jupe vers le bas, seringue toujours en place, et un bouchon 19 d'une prise d'air 20 est légèrement dévissé et le contenu du volume supérieur 9 est aspiré dans la seringue, le dispositif est remis en position droite, piston 5 vers le bas et jupe vers le haut et la seringue est alors dévissée, un premier filtre de porosité supérieure à 5 µm est positionné par connexion Luer sur ladite seringue connecté lui-même en ligne à un support de membrane contenant une membrane, deuxième filtre, de 0.2µm à 1µm de porosité, le contenu de la seringue passe séquentiellement au travers du premier filtre qui retient les agrégats plaquettaires et les globules blancs, et du deuxième filtre qui retient les micro-organismes. Le support du deuxième filtre est ouvert et la membrane est récupérée pour analyse des micro organismes retenus. Un exemple de support de filtre est donné dans le brevet US-2004/0208796.

Selon une deuxième réalisation, à la fin de l'incubation, la seringue positionnée à l'étape précédente est dévissée et un premier et un deuxième filtres en ligne sont insérés à sa place au niveau de la connexion Luer 15 protégée par la jupe, un premier filtre de porosité supérieur à 5 µm placé directement au contact de la jupe et un deuxième filtre de 0.2 µm à 1 µm de porosité inséré dans un support (exemple US-2004/0208796) et situé en ligne du premier filtre, le dispositif est retourné, piston 5 vers le haut et jupe vers le bas et le support de filtre est connecté à une source de vide, le bouchon 19 de la prise d'air 20 est légèrement dévissé, le vide est appliqué, le contenu du volume supérieur 9 passe séquentiellement au travers du premier filtre qui retient les agrégats plaquettaires et les globules blancs, et du deuxième filtre qui retient les micro-organismes. Le dispositif et le premier filtre sont détachés du support de filtre, le support du deuxième filtre est ouvert et la membrane est récupérée pour analyse des micro organismes retenus.

Avantageusement, il est possible de prélever 1 mL de filtrat à la fin de l'étape de filtration, pour cela, le dispositif est retourné, piston 5 vers le haut et jupe vers le bas seringue toujours en place, le bouchon 19 de la prise d'air 20 est légèrement dévissé et une partie du volume contenu dans le volume supérieur 9 est aspiré dans la seringue, le dispositif est remis en position droite, piston 5 vers le bas et jupe vers le haut et la seringue est alors dévissée et l'étape d'analyse peut se dérouler. Ce volume de filtrat contient les éventuels micro organismes intègres et pourra être utilisé comme échantillon destiné à l'antibiogramme.

L'intérêt du procédé et du dispositif décrits dans la présente invention est d'isoler et de concentrer les microorganismes d'un échantillon sanguin en quelques heures. Deux protocoles sont proposés, dans le premier, la première incubation dure 20 minutes et la deuxième incubation dure 4 heures à 35-37°C et dans le second protocole, appelé « protocole nuit » la première incubation dure plus de 12 heures à 35-37°C et la deuxième incubation dure 30 minutes. Les microorganismes sont isolés et concentrés en un temps minimum de 4 heures 20 minutes à partir d'un échantillon de 1 à 15 mL de slang total sur une membrane de filtration. Cette membrane peut alors être traitée par exemple pour une analyse de biologie moléculaire.

Les exemples proposés ont été réalisés avec des solutions de réactifs :
Première solution : 9 millilitres dans la chambre 4, elle est composée de réactifs accélérant la sédimentation des globules rouges et la croissance des bactéries:
   - TSB, Coeur-Cervelle (25%, 75%) ;
   - PEG 35 000 1% (w/v) ;
   - Lectin 6.66µg/mL ;
   - Eau osmosée.
Deuxième Solution : 1,2 millilitre ajouté dans la chambre 4, elle est composée de réactifs permettant l'agrégation des plaquettes et la croissance des bactéries :
   - TSB, Coeur-Cervelle (25%, 75%) ;
   - CD9 45mg / litre.

### Exemple 1 : Elimination des globules rouges et des plaquettes

3 à 5 millilitres de sang total sont prélevés dans le dispositif 1 contenant 9 millilitres de la première solution de réactifs comme décrit précédemment. Le dispositif est placé 30 minutes sur son socle 6 en position verticale à température ambiante. Le culot de globules rouges (GR) formé est isolé par action au niveau du piston 5. Le surnageant (SN) est récupéré. Une numération des globules rouges et des plaquettes dans le surnageant (GR dans SN et PLT dans SN) est effectuée avec un compteur cellulaire (Micros 60, ABX, France). Pour un des échantillons, la procédure est poursuivie par ajout de la deuxième solution de réactifs, incubation sous agitation pendant la nuit (environ 16 heures) puis filtration supérieure à 5µm de la totalité du surnageant. Une numération des plaquettes (PLT) dans le filtrat est effectuée avec un compteur cellulaire (Micros 60, ABX, France).

Les résultats sont résumés dans le tableau 1. Le nombre de GR dans l'échantillon (GR dans l'ech) est calculé en partant d'une concentration de GR théorique de 4x10⁶/µl. De même, le nombre de PLT (PLT dans l'ech) est calculé à partir d'une valeur théorique de 300x10³/µl. Après sédimentation et isolement des GR, le nombre de globules rouges résiduels dans le surnageant est divisé par 50 à 100 et le nombre de plaquettes résiduelles par 5: Après agrégation plaquettaire et filtration, ce nombre est divisé par 10.

Dans cet exemple, le procédé et le dispositif permettent une réduction de environ 2 LOG de globules rouges et de environ 1 LOG de plaquettes.

### Exemple 2 : récupération des micro organismes

Un échantillon de sang total est contaminé expérimentalement avec 4 souches bactériennes : Staphylococcus epidermidis (SE), Staphylococcus aureus (SA), Escherichia coli (EC) et Pseudomonas aeruginosa (PA) à environ 100 bactéries/ml. Un volume de sang est introduit dans le dispositif 1 et incubé dans la chambre 4 avec 9 mL de la première solution de réactifs. Le dispositif est placé 30 minutes sur son socle 6 en position verticale à température ambiante. Le culot de globules rouges formé est isolé par action au niveau du piston 5. Le surnageant (SN) est récupéré. La procédure est poursuivie par ajout de la deuxième solution de réactifs et incubation sous agitation 4 heures dans une enceinte thermorégulée à 35 - 37°C. Une numération de bactéries sur boite de pétri est réalisée en début de l'incubation à 35-37°C, à T0, et après 2 heures d'incubation, à T2H, et après 4 heures d'incubation, à T4H. Les colonies sur les boites de pétri sont comptées 24 à 48 heures après dépôt.

Les résultats sont présentés dans le tableau 2. L'incubation de 4H permet une croissance significative des bactéries dont le nombre est multiplié par 25 pour SE qui est connue en tant que bactérie de pousse lente, jusqu'à 9000 pour EC qui est connue en tant que bactérie de pousse rapide. Ces concentrations sont compatibles avec une méthode de détection en biologie moléculaire.

**Tableau 1**

| | Vol d'ech sanguin (mL) | GR dans l'ech | PLT dans l'ech | SN (m L) | GR dans le SN | PLT dans le SN | PLT dans le filtrat |
|---|---|---|---|---|---|---|---|
| | 4 | 16x 10⁹ | 12x 10⁸ | 7 | 2,8 x10 ⁸ | 4,3x1 0⁸ | |
| | 4 | 16x 10⁹ | 12x 10⁸ | 7 | 2,8 x10 8 | 5,6x1 0⁸ | |
| | 5 | 2x1 0¹⁰ | 15x 10⁸ | >7 | 2x1 0⁸ | 7,4x1 0⁸ | |
| | 4.5 | 18x 10⁹ | 14x 10⁸ | >7 | 2x1 0⁸ | 6,2x1 0⁸ | |
| | 3 | 12x 10⁹ | 9x1 0⁸ | 7 | 2x1 0⁸ | 1,2x1 0⁸ | |
| | 3.5 | 14x 10⁹ | 11x 10⁸ | 7 | 2x1 0⁸ | 3,5x1 0⁸ | 10⁸ |
| | 4 | 16x 10⁹ | 12x 10⁸ | 7 | 2x1 0⁸ | 3,3x1 0⁸ | |

**Tableau 2**

| | SE | SA | EC | PA |
|---|---|---|---|---|
| T0 | 40 | 60 | 130 | 450 |
| T2H | 220 | 350 | 1450 | 1020 |
| T4H | 1000 | 6000 | 11900 00 | 49000 |

Résultats exprimés en nombre de colonies / mL de surnageant

## Revendications

1. Dispositif de préparation d'un échantillon de fluide biologique en vue de son analyse bactériologique comprenant un récipient pourvu d'une chambre (4) dans laquelle un piston (5) est déplaçable entre une position d'ouverture et une position d'obturation, ledit dispositif étant **caractérisé en ce que** la chambre (4) comprend une zone de séparation (7) et un moyen d'introduction (15, 16) d'un fluide dans ladite chambre, ledit moyen d'introduction étant disposé dans la partie supérieure (2) de la chambre (4) et **en ce que** le piston (5) comprend un moyen d'obturation (8) coopérant avec la zone de séparation (7) de sorte à définir un volume supérieur (9) et un volume inférieur (10) de part et d'autre de ladite zone, le volume supérieur (9) et le volume inférieur (10) communiquant entre eux lorsque le piston (5) est en position d'ouverture et étant isolés l'un de l'autre de façon étanche lorsque le piston (5) est en position d'obturation, et ledit dispositif étant **caractérisé en ce que** la zone de séparation (7) comprend un goulot d'étranglement (18), le volume inférieur (10) s'étendant sous ledit goulot d'étranglement, le moyen d'obturation (8) étant agencé pour boucher ledit goulot lorsque le piston (5) est en position d'obturation de sorte à isoler le volume inférieur (10) du volume supérieur (9).

2. Dispositif de préparation selon la revendication 1, **caractérisé en ce que** le goulot (18) présente une forme annulaire sensiblement tronconique s'étendant dans le volume supérieur (9).

3. Dispositif de préparation selon la revendication 1 ou 2 **caractérisé en ce que** le piston (5) comprend une tige (11) s'étendant dans le volume inférieur (10), ledit volume inférieur s'étendant autour de ladite tige, ladite tige comprenant une protubérance (12) formant moyen d'obturation (8) prévue à sa partie extrême libre.

4. Dispositif de préparation selon l'une quelconque des revendications 1 à 3, **caractérisé en ce que** le récipient comprend une rainure de guidage (14), le piston (5) comprenant un ergot (19), ledit ergot étant disposé dans ladite rainure, le piston (5) se trouvant en position d'obturation lorsque l'ergot (19) est disposé à l'une des partie extrêmes de la rainure (14) et en position d'ouverture lorsque l'ergot (19) est disposé à l'autre partie extrême de la rainure (14).

5. Dispositif de préparation selon l'une quelconque des revendications 1 à 4, **caractérisé en ce que** le moyen d'introduction du fluide est formé par un connecteur Luer (15) disposé au-dessus de la chambre (4) et destiné à recevoir un récipient contenant le fluide à introduire dans ledit chambre.

6. Dispositif de préparation selon la revendication 5, **caractérisé en ce que** le moyen d'introduction du fluide comprend en outre un élément de réception (16) du récipient contenant le fluide à introduire, ledit élément étant associé à la partie supérieure (2) du récipient.

7. Dispositif de préparation selon l'une quelconque des revendications 1 à 6, **caractérisé en ce que** le récipient comprend une partie inférieure (3) et une partie supérieure (2), la zone de séparation (7) étant prévue dans la partie inférieure (3), lesdites parties étant associées l'une à l'autre de sorte à former la chambre (4).

8. Dispositif de préparation selon l'une quelconque des revendications 1 à 7, **caractérisé en ce que** la chambre (4) contient une première solution de réactifs agencée pour accélérer la sédimentation du fluide biologique et la croissance des microorganismes présents dans l'échantillon de fluide biologique à préparer.

9. Procédé de préparation d'un échantillon de fluide biologique en vue de son analyse bactériologique utilisant un dispositif de préparation (1) selon l'une quelconque des revendications 1 à 8, ledit procédé étant **caractérisé en ce qu'**il comprend les étapes consistant à:
- introduire le fluide biologique dans la chambre (4), le piston (5) étant en position d'ouverture;
- après sédimentation du fluide biologique, déplacer le piston (5) dans sa position d'obturation de sorte à séparer le culot de sédiment du surnageant, ledit culot se trouvant dans le volume inférieur (10) et ledit surnageant se trouvant dans le volume supérieur (9);
- mélanger le surnageant avec une solution de réactifs agencée pour favoriser la croissance des microorganismes présents dans ledit surnageant;
- prélever ledit surnageant en vue de son analyse bactériologique.

10. Procédé selon la revendication 9, **caractérisé en ce que** le fluide biologique est mélangé avec une première solution de réactifs agencée pour accélérer la sédimentation du fluide biologique et la croissance des microorganismes présents dans l'échantillon de fluide biologique lorsqu'il est introduit dans la chambre (4).

11. Procédé selon la revendication 9 ou 10, **caractérisé en ce qu'**il comprend une étape d'incubation et de filtration du surnageant après l'étape de mélange avec une solution de réactifs agencée pour favoriser la croissance des microorganismes.

12. Procédé selon l'une quelconque des revendications 9 à 11, **caractérisé en ce qu'**une quantité comprise entre 1 mL et 15 mL de fluide biologique est introduite dans la chambre (4).

13. Procédé selon l'une quelconque des revendications 9 à 12, **caractérisé en ce que** le fluide biologique est du sang total, le culot de sédiment étant des globules rouges.

## Patentansprüche

1. Vorrichtung zur Vorbereitung einer Probe einer biologischen Flüssigkeit im Hinblick auf ihre bakteriologische Analyse, umfassend einen Behälter, der mit einer Kammer (4) versehen ist, in der ein Kolben (5) zwischen einer geöffneten Position und einer geschlossenen Position verschiebbar ist, wobei die Vorrichtung **dadurch gekennzeichnet ist, dass** die Kammer (4) eine Trennungszone (7) und ein Mittel zur Einführung (15, 16) einer Flüssigkeit in die Kammer umfasst, wobei sich das Einführungsmittel im oberen Teil (2) der Kammer (4) befindet, und dass der Kolben (5) ein Verschlussmittel (8) umfasst, das so mit der Trennungszone (7) zusammenwirkt, dass ein oberes Volumen (9) und ein unteres Volumen (10) beiderseits der Trennungszone definiert wird, wobei das obere Volumen (9) und das untere Volumen (10) miteinander in Verbindung stehen, wenn sich der Kolben (5) in geöffneter Stellung befindet, und dicht voneinander isoliert sind, wenn sich der Kolben (5) in geschlossener Stellung befindet, und die Vorrichtung **dadurch gekennzeichnet ist, dass** die Trennungszone (7) einen Engpass (18) umfasst, wobei sich das untere Volumen (10) unter dem Engpass erstreckt, wobei das Verschlussmittel (8) vorgesehen ist, um den Engpass zu verstopfen, wenn sich der Kolben (5) in geschlossener Position befindet, so dass das untere Volumen (10) vom oberen Volumen (9) isoliert ist.

2. Vorrichtung zur Vorbereitung gemäß Anspruch 1, **dadurch gekennzeichnet, dass** der Engpass (18) eine im Wesentlichen kegelstumpfförmige Ringform aufweist, die sich im oberen Volumen (9) erstreckt.

3. Vorrichtung zur Vorbereitung gemäß Anspruch 1 oder 2, **dadurch gekennzeichnet, dass** der Kolben (5) eine Stange (11) umfasst, die sich im unteren Volumen (10) erstreckt, wobei sich das untere Volumen um die Stange herum erstreckt, wobei die Stange einen Fortsatz (12) umfasst, der das Verschlussmittel (8) bildet und an ihrem freien Endteil vorgesehen ist.

4. Vorrichtung zur Vorbereitung gemäß einem der Ansprüche 1 bis 3, **dadurch gekennzeichnet, dass** der Behälter eine Führungsnut (14) umfasst, wobei der Kolben (5) einen Vorsprung (19) umfasst, wobei sich der Vorsprung in der Nut befindet, wobei sich der Kolben (5) in der geschlossenen Position befindet, wenn sich der Vorsprung (19) an dem einen Endteil der Nut (14) befindet, und sich in der geöffneten Position befindet, wenn sich der Vorsprung (19) an dem anderen Endteil der Nut (14) befindet.

5. Vorrichtung zur Vorbereitung gemäß einem der Ansprüche 1 bis 4, **dadurch gekennzeichnet, dass** das Mittel zur Einführung der Flüssigkeit von einem Luer-Anschluss (15) gebildet wird, der sich über der Kammer (4) befindet und einen Behälter, der die in die Kammer einzuführende Flüssigkeit enthält, aufnehmen soll.

6. Vorrichtung zur Vorbereitung gemäß Anspruch 5, **dadurch gekennzeichnet, dass** das Mittel zur Einführung der Flüssigkeit weiterhin ein Element zur Aufnahme (16) des Behälters, der die einzuführende Flüssigkeit enthält, umfasst, wobei das Element mit dem oberen Teil (2) des Behälters verbunden ist.

7. Vorrichtung zur Vorbereitung gemäß einem der Ansprüche 1 bis 6, **dadurch gekennzeichnet, dass** der Behälter einen unteren Teil (3) und einen oberen Teil (2) umfasst, wobei die Trennungszone (7) im unteren Teil (3) vorgesehen ist, wobei die Teile so miteinander verbunden sind, dass sie die Kammer (4) bilden.

8. Vorrichtung zur Vorbereitung gemäß einem der Ansprüche 1 bis 7, **dadurch gekennzeichnet, dass** die Kammer (4) eine erste Lösung von Reagentien enthält, die dazu vorgesehen ist, die Sedimentation der biologischen Flüssigkeit und das Wachstum der Mikroorganismen, die in der vorzubereitenden Probe der biologischen Flüssigkeit vorhanden sind, zu beschleunigen.

9. Verfahren zur Vorbereitung einer Probe einer biologischen Flüssigkeit im Hinblick auf ihre bakteriologische Analyse unter Verwendung einer Vorrichtung zur Vorbereitung (1) gemäß einem der Ansprüche 1 bis 8, wobei das Verfahren **dadurch gekennzeichnet ist, dass** es die folgenden Schritte umfasst:
- Einführen der biologischen Flüssigkeit in die Kammer (4), wobei sich der Kolben (5) in geöffneter Position befindet;
- nach der Sedimentation der biologischen Flüssigkeit Verschieben des Kolbens (5) in seine geschlossene Position, um das Sediment vom Überstand zu trennen, wobei sich das Sediment im unteren Volumen (10) befindet und sich der Überstand im oberen Volumen (9) befindet;
- Mischen des Überstands mit einer Lösung von Reagentien, die dazu vorgesehen ist, das Wachstum der Mikroorganismen, die sich in dem Überstand befinden, zu fördern;
- Entnehmen des Überstands im Hinblick auf seine bakteriologische Analyse.

10. Verfahren gemäß Anspruch 9, **dadurch gekennzeichnet, dass** die biologische Flüssigkeit mit einer ersten Lösung von Reagentien, die dazu vorgesehen ist, die Sedimentation der biologischen Flüssigkeit und das Wachstum der Mikroorganismen, die in der Probe der biologischen Flüssigkeit vorhanden sind, wenn sie in die Kammer (4) eingeführt wird, zu beschleunigen, gemischt wird.

11. Verfahren gemäß Anspruch 9 oder 10, **dadurch gekennzeichnet, dass** es nach dem Schritt des Mischens mit einer Lösung von Reagentien, die dazu vorgesehen ist, das Wachstum der Mikroorganismen zu beschleunigen, einen Schritt der Inkubation und Filtration des Überstands umfasst.

12. Verfahren gemäß einem der Ansprüche 9 bis 11, **dadurch gekennzeichnet, dass** eine Menge der biologischen Flüssigkeit im Bereich von 1 ml bis 15 ml in die Kammer (4) eingeführt wird.

13. Verfahren gemäß einem der Ansprüche 9 bis 12, **dadurch gekennzeichnet, dass** es sich bei der biologischen Flüssigkeit um Vollblut und bei dem Sediment um rote Blutkörperchen handelt.

## Claims

1. A device for preparation of a sample of biological fluid for its bacteriological analysis and comprising a container provided with a chamber (4) in which a piston (5) is movable between an open position and closing position, and said device being **characterized in that** the chamber (4) comprises a separation zone (7) and a means for introduction (15, 16) of a fluid into said chamber, said means of introduction being arranged in the upper part (2) of the chamber (4), and **in that** the piston (5) comprises a closure means (8) cooperating with the separation zone (7) in order to define an upper volume (9) and a lower volume (10) on both sides of said zone, the upper volume (9) and the lower volume (10) communicating with each other when the piston (5) is in the open position and tightly isolated from each other when the piston (5) is in the closing position, and said device being **characterized in that** the separation zone (7) comprises a bottleneck (18), the lower volume (10) extending under said bottleneck, the closure means (8) being constructed to close said bottleneck when the piston (5) is in the closing position in order to isolate the lower volume (10) from the upper volume (9).

2. The device for preparation according to claim 1, **characterized in that** the bottleneck (18) has a substantially truncated cone-shaped annular shape extending into the upper volume (9).

3. The device for preparation according to claim 1 or 2, **characterized in that** the piston (5) comprises a rod (11) extending into the lower volume (10), said lower volume extending around said rod, said rod comprising a protuberance (12) forming closure means (8) provided in its free end part.

4. The device for preparation according to any of claims 1 to 3, **characterized in that** the container comprises a guiding groove (14), the piston (5) comprising a pin (19) arranged in said groove, the piston (5) being found in the closing position when the pin (19) is arranged at one of the end parts of the groove (14), and in the open position when the pin (19) is arranged in the other end part of the groove (14).

5. The device for preparation according to any of claims 1 to 4, **characterized in that** the means for introduction of the fluid is formed by a Luer connector (15) arranged above the chamber (4) and designed to receive a container containing the fluid to be introduced into said chamber.

6. The device for preparation according to claim 5, **characterized in that** the means for introduction of the fluid further comprises a receiving part (16) of the container containing the fluid to be introduced, said part being associated with the upper part (2) of the container.

7. The device for preparation according to any of claims 1 to 6, **characterized in that** the container comprises a lower part (3) and an upper part (2), the separation zone (7) being provided in the lower part (3), said parts being associated with each other so as to form the chamber (4).

8. The device for preparation according to any of claims 1 to 7, **characterized in that** the chamber (4) contains a first reagent solution constricted to accelerate the sedimentation of the biological fluid and the growth of the microorganisms present in the sample of biological fluid to be prepared.

9. A method for preparation of a sample of biological fluid for its bacteriological analysis using a device for preparation (1) according to any of claims 1 to 8, said method being **characterized in that** it is comprised of steps consisting of:
- introducing the biological fluid into the chamber (4), the piston (5) being in the open position;
- after sedimentation of the biological fluid, moving the piston (5) to its closing position in order to separate the sediment pellet from the supernatant, said pellet being found in the lower volume (10) and said supernatant being found in the upper volume (9);
- mixing the supernatant with a solution of reagents constructed to promote the growth of microorganisms present in said supernatant;
- sampling said supernatant for its bacteriological analysis.

10. The method according to claim 9, **characterized in that** the biological fluid is mixed with a first reagent solution constructed to accelerate the sedimentation of the biological fluid and the growth of the microorganisms present in the sample of biological fluid when it is introduced into the chamber (4).

11. The method according to claim 9 or 10, **characterized in that** it further comprises a step of incubation and filtration of the supernatant after the mixing step with a reagent solution constructed to promote the growth of microorganisms.

12. The method according to any of claims 9 to 11, **characterized in that** a quantity between 1 ml and 15 ml of biological fluid is introduced into the chamber (4).

13. The method according to any of claims 9 to 12, **characterized in that** the biological fluid is whole blood, the sediment pellet being red blood cells.
